# FASCICULE DE BREVET EUROPEEN

(11) **EP 1 056 408 B1**
(45) Date de publication et mention de la délivrance du brevet: **17.12.2003**
(21) Numéro de dépôt: 99904916.6
(22) Date de dépôt: 19.02.1999
(51) Int. Cl.: A61B 17/70, A61B 17/82, A61B 17/84

(54) **PROTHESE INTER-EPINEUSE**
ZWISCHEN WIRBELDORNFORTSÄTZEN EINGESETZTE PROTHESE
INTERSPINOUS PROSTHESIS

(30) Priorité: 20.02.1998 FR 9802300
(43) Date de publication de la demande: 06.12.2000
(73) Titulaire: Taylor, Jean, 06400 Cannes (FR)
(72) Inventeur: Taylor, Jean, 06400 Cannes (FR)
(74) Mandataire: Bratel, Gérard
(86) Numéro de dépôt international: FR9900383
(87) Numéro de publication internationale: WO99042051

(56) Documents cités:
- EP-A- 0 322 334
- DE-C- 3 113 142
- FR-A- 2 623 085
- FR-A- 2 717 675
- FR-A- 2 730 156
- US-A- 4 570 618

## Description

La présente invention concerne une prothèse inter-épineuse, destinée à produire une assistance discale et à amortir les mouvements relatifs de deux vertèbres adjacentes lors des mouvements de flexion ou d'extension du rachis.

Le brevet français n° 2717675, déposé au nom du demandeur, décrit une prothèse comprenant un corps en matière souple et deux inserts rigides en forme de V. Le corps est destiné à être inséré entre les apophyses épineuses de deux vertèbres pour maintenir un écartement anatomique souple entre celles-ci, tout en autorisant leur mouvement relatif. Les inserts permettent de délimiter des rainures pour recevoir les apophyses épineuses des deux vertèbres et comprennent un moyen pour fixer la prothèse à ces apophyses.

La prothèse selon ce brevet antérieur donne satisfaction en pratique, mais il est apparu qu'elle pouvait être améliorée, notamment en ce qui concerne sa structure et sa résistance aux contraintes répétées qu'elle subit. Les liaisons entre le corps de la prothèse et les inserts sont en effet très sollicitées.

La demande de brevet français n° 2 623 085 décrit une cale à structure en forme de H qui présente, sur l'une seulement de ses deux faces d'extrémité, ou sur les deux, une gorge dimensionnée pour recevoir, avec un léger jeu latéral, une apophyse épineuse respective. Chaque gorge est délimitée par des lèvres de faible hauteur qui, vues de côté, ont une forme en arc de cercle.

La présente invention a pour objet de fournir une prothèse inter-épineuse assurant un parfait soutien des deux vertèbres concernées, et permettant par conséquent une parfaite assistance discale, cette prothèse devant pouvoir résister aux contraintes répétées exercées sur elle par les apophyses, devant être conformée de manière à ne subir qu'une usure faible et devant présenter une stabilité importante dans toutes les directions, en particulier vis-à-vis des mouvements de "bascule latérale", c'est-à-dire des mouvements provoqués par la torsion du rachis selon son axe.

La prothèse objet de la présente demande de brevet est, de manière connue en soi, réalisée en un matériau multidirectionnellement souple et élastique, et comprend une portion inter-épineuse ayant une épaisseur légèrement supérieure à l'espace inter-épineux anatomique lorsque le rachis est en lordose, de telle sorte que cette portion est légèrement comprimée lorsque la prothèse est placée entre les apophyses épineuses de deux vertèbres.

Selon l'invention, la prothèse présente deux paires d'oreilles faisant saillie longitudinalement de part et d'autre de sa portion inter-épineuse, et ayant des hauteurs importantes par rapport à la hauteur totale de la prothèse, de l'ordre, pour chaque paire d'oreilles, de 30 à 45 % de cette hauteur totale, chaque paire d'oreilles faisant corps avec ladite portion inter-épineuse et délimitant un évidement profond apte à recevoir sans jeu l'apophyse épineuse correspondante, avec une large surface de contact de ces oreilles et de cette apophyse.

Ces évidements profonds assurent un parfait maintien de la prothèse en position entre les apophyses épineuses des vertèbres, dans toutes les directions, et en particulier vis-à-vis des mouvements résultant d'une torsion du rachis selon son axe, qui tendent à créer une bascule latérale de la prothèse.

De plus et surtout, la combinaison de cette structure en matériau multidirectionnellement souple et élastique, d'une part, et de ces deux paires d'oreilles de longueur importante et formant corps avec la portion inter-épineuse de la prothèse, d'autre part, permet, lorsque cette portion inter-épineuse est mise en compression, d'amener les oreilles en appui contre les faces latérales des apophyses épineuses, par un effet d' "auto-serrage".

Ce maintien de la prothèse, allié à la souplesse de cette prothèse, permet de réduire notablement les frottements entre la prothèse et les apophyses, ce qui rend inutiles les inserts en matériau rigide de la prothèse selon la technique antérieure. La prothèse selon l'invention est donc monobloc, ce qui résoud en outre les problèmes liés à la fabrication et surtout à la résistance dans le temps de la prothèse selon la technique antérieure.

La prothèse selon l'invention conjugue un effet de suppression des contacts nouvellement créés entre les facettes à la suite d'appuis intenses et un effet de réduction de la pression intra-discale, permettant un ralentissement du vieillissement discal.

Les indications principales de cette prothèse sont :
- arthropathie des facettes ;
- prévention de la dégénérescence discale se produisant consécutivement à une arthrodèse ;
- "soulagement" de l'annulus discal subsistant après traitement chirurgical d'une hernie discale.

Les faces internes de deux oreilles d'une même paire d'oreilles sont de préférence inclinées de manière à converger l'une vers l'autre en direction du fond de l'évidement qu'elles délimitent.

Un relatif coincement de l'apophyse est ainsi obtenu par légère déformation élastique des oreilles, qui contribue au maintien de la prothèse par rapport aux apophyses.

Les oreilles ont avantageusement une épaisseur moyenne relativement importante par rapport à la largeur moyenne de la prothèse, de l'ordre, pour chaque oreille, de 25 à 35 % de cette largeur moyenne.

Ces oreilles font ainsi parfaitement corps avec la portion inter-épineuse de la prothèse, ce qui assure leur résistance aux contraintes répétées subies par celle-ci.

De préférence, la face antérieure de la prothèse se raccorde respectivement aux faces supérieure et inférieure de la prothèse par des zones taillées de biais et/ou arrondies, permettant l'effacement total des angles que formeraient sinon ces faces deux à deux.

La prothèse peut ainsi être placée au niveau de la base des apophyses épineuses, à la jonction lame-épineuse des vertèbres, ce qui réduit l'amplitude des contraintes en torsion que les apophyses sont susceptibles d'exercer sur elle.

Avantageusement, la prothèse est percée d'au moins un conduit transversal aménagé au niveau de sa portion inter-épineuse, ce conduit permettant l'engagement d'un lien destiné à relier étroitement la prothèse à au moins une des apophyses épineuses.

Ces liens ont simplement pour objet de sécuriser la mise en place de la prothèse, celle-ci étant, par sa structure précitée, auto-serrée entre les apophyses.

De préférence, la paroi de la portion inter-épineuse qui délimite ce conduit est évasée au niveau des extrémités de ce conduit, pour éliminer toute arête susceptible de créer un point d'usure dudit lien.

Selon une forme de réalisation préférée de l'invention dans ce cas, la prothèse comprend deux conduits transversaux recevant chacun un lien pour la relier à l'apophyse épineuse de la vertèbre correspondante.

La prothèse selon l'invention est avantageusement placée dans une gaine textile qui épouse sa forme.

Cette gaine évite le contact direct du matériau synthétique qui la constitue, notamment du silicone, avec les tissus environnants, et facilite l'intégration de la prothèse à ces tissus. En outre, elle constitue un moyen de limitation de l'étirement de la prothèse, éliminant tout risqué de rupture de celle-ci en cas de charge exceptionnellement élevée.

Avantageusement, cette gaine comprend une bandelette cousue à elle sur le côté postérieur de la prothèse, qui peut servir de point d'ancrage à un ligament prothétique de substitution du ligament inter- et supra-épineux.

Pour sa bonne compréhension, l'invention est à nouveau décrite ci-dessous en référence au dessin schématique annexé représentant, à titre d'exemple non limitatif, une forme de réalisation préférée de la prothèse inter-épineusé qu'elle concerne.
La figure 1 est une vue en perspective d'une pièce en silicone que comprend cette prothèse ;
les figures 2 et 3 sont des vues en coupe de cette pièce selon respectivement les lignes II-II et III-III de la figure 1, et
les figures 4 et 5 sont des vues en perspective de la prothèse au cours de deux phases d'implantation sur des vertèbres.

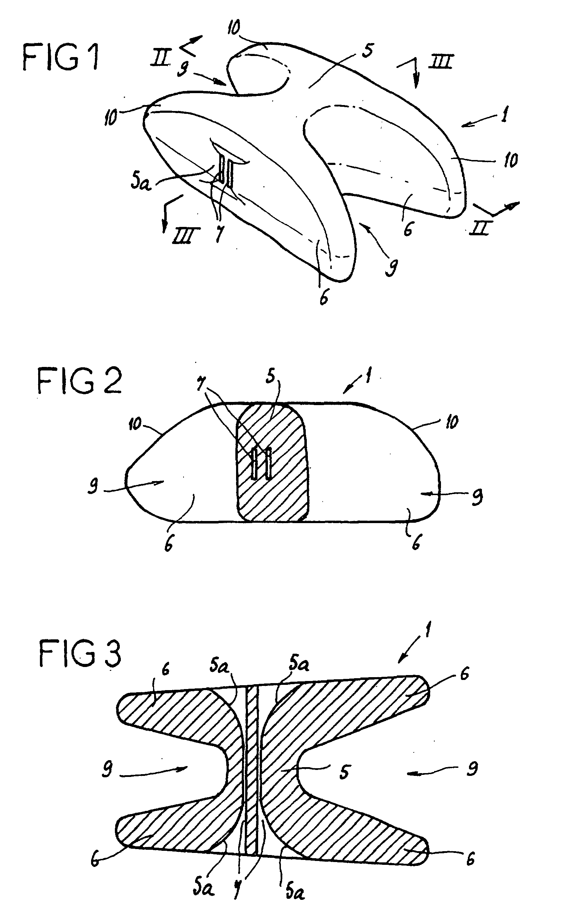

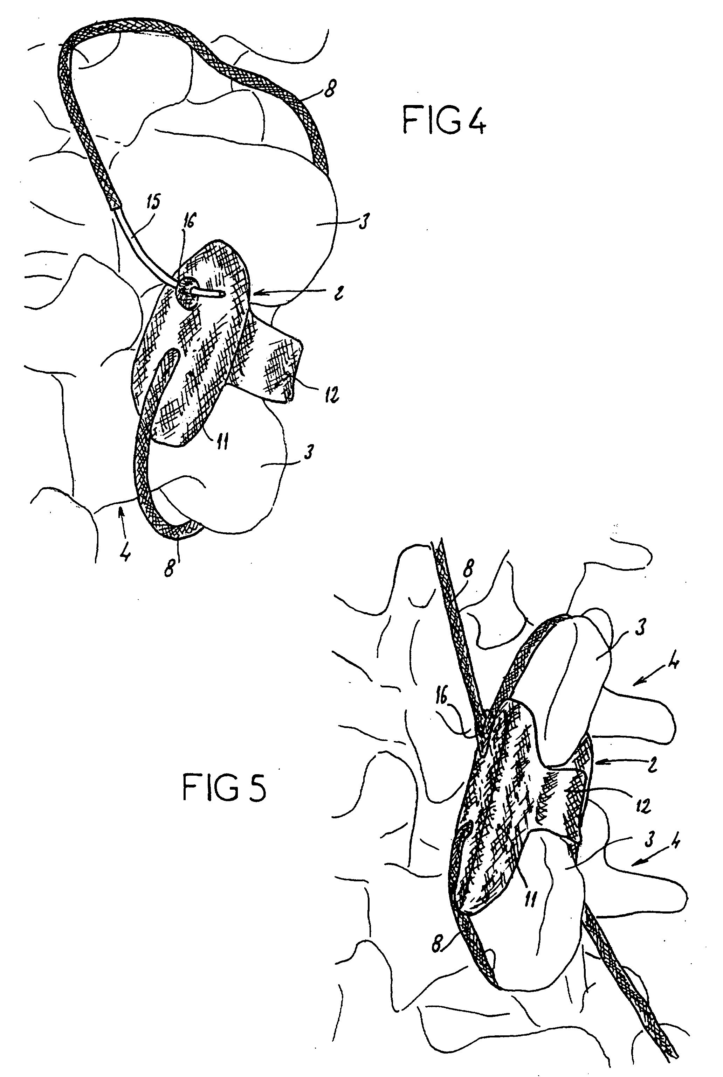

Les figures 1 à 3 représentent, sous différents angles, une pièce 1 en silicone constituant le noyau d'une prothèse inter-épineuse 2. Ainsi que cela apparaît aux figures 4 et 5, cette prothèse 2 est destinée à être placée entre les apophyses épineuses 3 de deux vertèbres adjacentes 4 pour amortir les mouvements relatifs de ces vertèbres 4 lors des mouvements de flexion ou d'extension du rachis.

La pièce 1 comprend une portion inter-épineuse 5 et deux paires d'oreilles latérales 6 faisant saillie longitudinalement de part et d'autre de cette portion 5.

La portion 5 a une épaisseur légèrement supérieure à l'espace inter-épineux anatomique lorsque le rachis est en lordose ; elle est donc légèrement comprimée lorsque la prothèse 2 est placée entre les apophyses 3.

Cette portion 5 est percée de deux conduits transversaux 7 permettant, ainsi que le montrent les figures 4 et 5, l'engagement de deux liens 8 qui servent à relier étroitement la prothèse 2 à chacune des apophyses 3. La paroi 5a de la portion 5 qui délimite chacun de ces conduits 7 du côté des oreilles 6 correspondantes est évasée au niveau des extrémités du conduit 7, pour éliminer toute arête susceptible de créer un point d'usure du lien 8.

Les oreilles 6 ont des hauteurs importantes par rapport à la hauteur totale de la prothèse 2, de l'ordre, pour les oreilles supérieures et inférieures, de 33 % et de 40 % de cette hauteur totale, respectivement.

Les faces internes de deux oreilles 6 d'une même paire d'oreilles sont inclinées de manière à converger l'une vers l'autre en direction du fond de l'évidement 9 qu'elles délimitent entre elles ; dans l'exemple montré au dessin, l'angle formé par les deux parois des oreilles supérieures est de l'ordre de 27°, tandis que l'angle formé par les deux parois des oreilles inférieures est de l'ordre de 43°.

Les oreilles 6 ont en outre une épaisseur moyenne relativement importante par rapport à la largeur moyenne de la prothèse 2, de l'ordre, pour les oreilles supérieures et inférieures, de 27 % et de 30 % de cette largeur moyenne, respectivement.

Il apparaît en outre aux figures 1 et 2 que la face antérieure de la pièce 1 se raccorde respectivement aux faces supérieure et inférieure de celle-ci par des zones 10 taillées de biais ou arrondies, permettant l'effacement total des angles que formeraient sinon ces faces antérieure et supérieure ou inférieure entre elles.

La pièce 1 est placée dans une gaine textile 11, en polyester, qui épouse sa forme et qui est percée de trous en correspondance des ouvertures des conduits 7.

Cette gaine 11 comprend une bandelette 12 cousue à elle sur le côté postérieur de la prothèse 2, destinée à servir de point d'ancrage à un ligament prothétique de substitution du ligament inter- et supra-épineux.

Il apparaît aux figures 4 et 5 que chaque lien 8 est constitué par une tresse dont une extrémité est sertie sur l'extrémité d'une aiguille courbe 15 et dont l'autre extrémité comporte un anneau 16.

En pratique, la prothèse 2 est insérée dans l'espace inter-épineux destiné à la recevoir. Grâce à ses zones 10, elle peut être insérée dans le fond de cet espace, jusqu'à la jonction entre les lames et les épineuses des vertèbres 4.

Chaque lien 8 est introduit dans le conduit 7 qui lui correspond, puis est engagé, grâce à l'aiguille 15, autour de l'apophyse épineuse 3 correspondante, puis au travers de l'anneau 16. Après mise en tension adéquate du lien 8, une pièce d'arrêt (non représentée), comprenant une collerette d'arrêt et un manchon susceptible d'être serti autour du lien 8, est engagée sur ce lien 8 jusqu' à ce que ladite collerette vienne en appui contre l'anneau 16. Ledit manchon est alors serti sur le lien 8 pour assurer la fixation de ce lien, et l'extrémité libre inutilisée du lien 8 est coupée au ras de ce manchon.

Il va de soi que l'invention n'est pas limitée à la forme de réalisation décrite ci-dessus à titre d'exemple mais qu'elle en embrasse, au contraire, toutes les variantes de réalisation. Ainsi, la pièce 1 pourrait être utilisée seule, sans la gaine 11, cette pièce 1 constituant alors elle-même la prothèse selon l'invention.

## Revendications

1. Prothèse inter-épineuse, réalisée en un matériau multidirectionnellement souple et élastique, et comprenant une portion inter-épineuse (5) ayant une épaisseur légèrement supérieure à l'espace inter-épineux anatomique lorsque le rachis est en lordose, de telle sorte que cette portion (5) est légèrement comprimée lorsque la prothèse (2) est placée entre les apophyses épineuses (3) de deux vertèbres (4), prothèse (2) **caractérisée en ce qu'**elle présente deux paires d'oreilles (6) faisant saillie longitudinalement de part et d'autre de sa portion inter-épineuse (5), ces oreilles (6) ayant des hauteurs importantes par rapport à la hauteur totale de la prothèse (2), de l'ordre, pour chaque paire d'oreilles (6), de 30 à 45 % de cette hauteur totale ; chaque paire d'oreilles (6) fait corps avec ladite portion inter-épineuse (5) et délimite un évidement profond (9) apte à recevoir sans jeu l'apophyse épineuse (3) correspondante, avec une large surface de contact de ces oreilles (6) et de cette apophyse (3).

2. Prothèse selon la revendication 1, **caractérisée en ce que** les faces internes de deux oreilles (6) d'une même paire d'oreilles sont inclinées de manière à converger l'une vers l'autre en direction du fond de l'évidement (9) qu'elles délimitent.

3. Prothèse selon la revendication 1 ou la revendication 2, **caractérisée en ce que** les oreilles (6) ont une épaisseur moyenne relativement importante par rapport à la largeur moyenne de la prothèse (2), de l'ordre, pour chaque oreille (6), de 25 à 35 % de cette largeur moyenne.

4. Prothèse selon l'une des revendications 1 à 3, **caractérisée en ce que** sa face antérieure se raccorde respectivement à ses faces supérieure et inférieure par des zones (10) taillées de biais et/ou arrondies, permettant l'effacement total des angles que formeraient sinon ces faces deux à deux.

5. Prothèse selon l'une des revendications 1 à 4, **caractérisée en ce qu'**elle est percée d'au moins un conduit transversal (7) aménagé au niveau de sa portion inter-épineuse (5), ce conduit (7) permettant l'engagement d'un lien (8) destiné à relier étroitement la prothèse (2) à au moins une des apophyses épineuses (3).

6. Prothèse selon la revendication 5, **caractérisée en ce que** la paroi (5a) de la portion inter-épineuse (5) qui délimite ledit conduit (7) est évasée au niveau des extrémités de ce conduit (7), pour éliminer toute arête susceptible de créer un point d'usure dudit lien (8).

7. Prothèse selon la revendication 5 ou la revendication 6, **caractérisée en ce qu'**elle comprend deux conduits transversaux (7) recevant chacun un lien (8) pour la relier à l'apophyse épineuse (3) de la vertèbre (4) correspondante.

8. Prothèse selon l'une des revendications 1 à 7, **caractérisée en ce qu'**elle est placée dans une gaine textile (11) qui épouse sa forme.

9. Prothèse selon la revendication 8, **caractérisée en ce que** la gaine (11) comprend une bandelette (12) cousue à elle sur le côté postérieur de la prothèse, destinée à servir de point d'ancrage à un ligament prothétique de substitution du ligament inter- et supra-épineux.

## Claims

1. An interspinous prosthesis made of a multidirectionally flexible and resilient material and comprising an interspinous portion (5) slightly thicker than the anatomical interspinous space when the rachis is lordotic, in such a way that said portion (5) is slightly compressed when the prosthesis (2) is positioned between the spinous apophyses (3) of two vertebrae (4), said prosthesis (2) being **characterised in that** it has two pairs of lugs (6) projecting longitudinally either side of its interspinous portion (5), said lugs (6) exhibiting considerable heights relative to the total height of the prosthesis (2), of the order, for each pair of lugs (6), of 30 to 45 % of said total height; each pair of lugs (6) is integral with said interspinous portion (5) and defines a deep recess (9) capable of receiving without play the corresponding spinous apophysis (3), with a wide contact surface between said lugs (6) and said apophysis (3).

2. A prosthesis according to claim 1, **characterised in that** the inner faces of two lugs (6) of one and the same pair of lugs are inclined in such a way as to converge towards one another in the direction of the base of the recess (9) which they define.

3. A prosthesis according to claim 1 or claim 2, **characterised in that** the lugs (6) exhibit a relatively large average thickness relative to the average width of the prosthesis (2), of the order, for each lug (6), of 25 to 35 % of said average width.

4. A prosthesis according to one of claims 1 to 3, **characterised in that** its anterior face is connected respectively to its upper and lower faces by bevelled and/or rounded zones (10), allowing the total elimination of the angles which said pairs of faces would otherwise form.

5. A prosthesis according to one of claims 1 to 4, **characterised in that** at least one transverse conduit (7) is formed therein at the level of its interspinous portion (5), said conduit (7) allowing the engagement of a tie (8) designed to connect the prosthesis (2) closely to at least one of the spinous apophyses (3).

6. A prosthesis according to claim 5, **characterised in that** the wall (5a) of the interspinous portion (5) which defines said conduit (7) is flared at the level of the ends of said conduit (7), to eliminate any edge liable to create a point of wear on said tie (8).

7. A prosthesis according to claim 5 or claim 6, **characterised in that** it comprises two transverse conduits (7) each receiving a tie (8) for connecting it to the spinous apophysis (3) of the corresponding vertebra (4).

8. A prosthesis according to one of claims 1 to 7, **characterised in that** it is placed in a textile sheath (11) which conforms to its shape.

9. A prosthesis according to claim 8, **characterised in that** the sheath (11) comprises a small strip (12) sewn thereto on the posterior side of the prosthesis, designed to serve as an anchoring point for a prosthetic ligament replacing the inter- and supraspinous ligament.

## Patentansprüche

1. Interspinale Prothese aus einem multidirektional nachgiebigen und elastischen Material mit einem interspinalen Abschnitt (5), der eine geringfügig grössere Dicke am anatomischen interspinalen Raum hat, wenn der Rachis eine Lordose aufweist, so dass dieser Abschnitt (5) leicht komprimiert wird, wenn die Prothese (2) zwischen die spinalen Apophysen (3) zweier Wirbel (4) gebracht wird, wobei die Prothese (2) **dadurch gekennzeichnet ist, dass** sie zwei Ohrenpaare (6) aufweist, die beiderseits ihres interspinalen Abschnitts (5) in Längsrichtung hervorstehen, wobei diese Ohren (6) bezüglich der Gesamthöhe der Prothese (2) grosse Höhen von etwa 30 bis 45% dieser Gesamthöhe für jedes Ohrenpaar (6) haben; wobei jedes Ohrenpaar (6) mit dem interspinalen Abschnitt (5) einstückig ist und eine tiefe Aussparung (9) begrenzt, welche die entsprechende spinale Apophyse (3) ohne Spiel und mit einer grossen Berührungsfläche zwischen diesen Ohren (6) und dieser Apophyse (3) aufnehmen kann.

2. Prothese nach Anspruch 1, **dadurch gekennzeichnet, dass** die Innenflächen der beiden Ohren (6) eines Ohrenpaares derart geneigt sind, dass sie zur Richtung des Bodens der durch sie begrenzten Aussparung (9) zueinander konvergieren.

3. Prothese nach Anspruch 1 oder 2, **dadurch gekennzeichnet, dass** die Ohren (6) eine relativ grosse mittlere Dicke bezüglich der mittleren Breite der Prothese (2) von etwa 25 bis 35% dieser mittleren Breite für jedes Ohr (6) haben.

4. Prothese nach einem der Ansprüche 1 bis 3, **dadurch gekennzeichnet, dass** diese vordere Fläche sich an die obere und untere Seite bezüglich der abgeschrägten und/oder abgerundeten Bereiche (10) anschliesst, wodurch eine vollständige Vermeidung der Winkel ermöglicht wird, die sonst diese Flächen zu zweit bilden würden.

5. Prothese nach einem der Ansprüche 1 bis 4, **dadurch gekennzeichnet, dass** sie von mindestens einem quer verlaufenden Kanal (7) durchdrungen ist, der bei dem interspinalen Abschnitt (5) angeordnet ist, wobei dieser Kanal (7) den Eingriff eines Verbindungsmittels (8) ermöglicht, das dazu bestimmt ist, die Prothese (2) mit mindestens einer der spinalen Apophysen (3) eng zu verbinden.

6. Prothese nach Anspruch 5, **dadurch gekennzeichnet, dass** die Wand (5a) des interspinalen Abschnitts (5), welche den Kanal (7) begrenzt, an den Enden dieses Kanals (7) aufgeweitet ist, um jegliche Kante zu beseitigen, die einen Abnutzungspunkt des Verbindungsmittels (8) erzeugen könnte.

7. Prothese nach Anspruch 5 oder 6, **dadurch gekennzeichnet, dass** sie zwei quer verlaufende Kanäle (7) aufweist, von denen jeder ein Verbindungsmittel (8) aufnimmt, um sie mit der spinalen Apophyse (3) des entsprechenden Wirbels (4) zu verbinden.

8. Prothese nach einem der Ansprüche 1 bis 7, **dadurch gekennzeichnet, dass** sie in eine Textilumhüllung (11) eingebracht ist, die sich an ihre Form anschmiegt.

9. Prothese nach Anspruch 8, **dadurch gekennzeichnet, dass** die Umhüllung (11) ein Bändchen (12) aufweist, das an ihr auf der hinteren Seite der Prothese angenäht ist und als Verankerungspunkt für ein prothetisches Band als Ersatz des inter- und supraspinalen Bandes dient.
